# EUROPEAN PATENT SPECIFICATION

(11) **EP 1 343 474 B1**
(45) Date of publication and mention of the grant of the patent: **03.03.2010**
(21) Application number: 01987379.3
(22) Date of filing: 11.12.2001
(51) Int. Cl.: A61K 9/08, A61P 27/04

(54) **INTRAOCULAR IRRIGATING SOLUTION HAVING IMPROVED FLOW CHARACTERISTICS**
INTRAOCULARE IRRIGATIONSLÖSUNG MIT VERBESSERTEN FLIESSEIGENSCHAFTEN
SOLUTIONS POUR IRRIGATIONS INTRAOCULAIRES A FLUIDITE AMELIOREE

(30) Priority: 20.12.2000 US 257570 P
(43) Date of publication of application: 17.09.2003
(73) Proprietor: Alcon, Inc., 6331 Hünenberg (CH)
(72) Inventor: SHAH, Mandar, V., Arlington, TX 76016 (US); BOUKHNY, Mikhail, Laguna Niguel, CA 92677 (US); GARNER, William, H., Southlake, TX 76092 (US); MARKWARDT, Kerry, L., Mansfield, TX 76063 (US); DOSHI, Uday, Randolph, New Jersey 07869 (US)
(74) Representative: Best, Michael
(86) International application number: PCT/US2001/048094
(87) International publication number: WO 2002/049614

(56) References cited:
- EP-A- 0 517 970
- EP-A- 0 938 903
- GB-A- 2 204 238
- US-A- 4 238 482
- US-A- 5 895 645
- ASSIA EI ET AL.: "Experimental studies on viscofluids for intraocular surgery" JOURNAL OF CATARACT AND REFRACTIVE SURGERY, vol. 24, no. 1, 1998, pages 78-83, XP008006361 ISSN: 0886-3350
- HUANG EC ET AL.: "Accomodative microfluctuations and iris contour" JOURNAL OF VISION, vol. 6, 2006, pages 653-660,
- SILVER DM ET AL.: "Aqueous flow through the iris-lens channel: estimates of differential pressure bewteen the anterior and posterior chambers" JOURNAL OF GLAUCOMA, vol. 13, 2004, pages 100-107,
- MOSES RA: "Circumferential flow in Schlemm's canal" AMERICAN JOURNAL OF OPHTHALMOLOGY, vol. 88, 1979, pages 585-591,
- JAMES CJ ET AL.: "Viscometer calibration standards: viscosities of water between 0 and 60°C and of selected aqueous sucrose solutions at 25°C from measurements with a flared capillary viscometer" JOURNAL OF PHYSICS D: APPLIED PHYSICS, vol. 17, 1984, pages 225-230,
- BESWICK JA ET AL.: "Effect of hyaluronidase on the viscosity of the aqueous humour" BRITISH JOURNAL OF OPHTHALMOLOGY, vol. 40, 1956, pages 545-548, XP008079510
- FECHNER PU AND FECHNER MU: "Methylcellulose and lens implantation" BRITISH JOURNAL OF OPHTHALMOLOGY, vol. 67, 1983, pages 259-263, XP008079635
- FLEMING TC ET AL.: "Studies on the irritating action of methylcellulose" ARCHIVES OF OPHTHALMOLOGY, vol. 61, 1959, pages 565-567, XP008079731
- MUELLER WH ET AL.: "Ophthalmic vehicles: the effect of methylcellulose on the penetration of homatropine hydrobromide through the cornea" JOURNAL OF THE AMERICAN PHARMACEUTICAL ASSOCIATION (SCIENTIFIC EDITION), vol. 45, 1956, pages 334-341,

## Description

### Background of the Invention

The present invention is directed to the field of intraocular surgery. More specifically, the invention is directed to the irrigation of intraocular tissues during cataract surgery, vitrectomy surgery, and other intraocular surgical procedures. The invention provides intraocular irrigating solutions that have improved physical properties (e.g., flow characteristics) relative to prior ophthalmic irrigating solutions.

The field of intraocular surgery has advanced dramatically over the past twenty years. The advancements in this art have resulted from significant improvements in the areas of surgical techniques, surgical equipment and associated pharmaceutical products. Despite these advancements, intraocular surgery is still a very delicate process with little room for error and great potential for harm to both ocular tissues and, ultimately, the vision of the patient. Thus, there is an ongoing need to improve ophthalmic surgical techniques and equipment, as well as associated pharmaceutical products.

The present invention has resulted from an effort to improve the fluid dynamics of intraocular irrigating solutions, so as to provide greater protection for delicate intraocular tissues, while at the same time enhancing the ability of ophthalmic surgeons to perform surgical procedures more efficiently.

Although various techniques have been used previously to remove the natural crystalline lens of the eye when it becomes afflicted with a cataract, the majority of cataract surgeries today are performed by using a procedure known as "phacoemulsification". This procedure involves the use of a surgical handpiece having a tip that vibrates at an ultrasonic frequency. The vibrating tip of the handpiece is utilized to disintegrate or "emulsify" the cataractous lens. This process necessarily generates lens fragments or particles within the eye that can cause irreparable physical damage to corneal endothelial cells if those cells are left unprotected. The corneal endothelial cells are normally protected during the phacoemulsification procedure by injecting a viscoelastic material (e.g., hyaluronic acid) into the eye to form a protective barrier over the corneal endothelial cells. However, even with the presence of the viscoelastic material, lens particles continue to move in the eye, particularly when the viscoelastic material is removed by a combined irrigating/aspiration handpiece following the phacoemulsification of the lens, prior to insertion of an artificial lens.

Due to continuous irrigation and aspiration, usually there is a lot of turbulence in the anterior chamber, within which non-aspirated lens fragments move around. In addition, the ultrasonic vibrations produced by the tip of the phacoemulsification handpiece push the lens fragments away from the tip thereby making it difficult to aspirate the fragments via the aspiration line in the tip of the handpiece. The movement of these lens fragments can cause damage to the surrounding tissue.

In addition to the lens fragments, damage may result directly from the turbulent flow of fluids intraocularly or from bubbles generated in the intraocular fluids by the phacoemulsification handpiece. Air bubbles generated during intraocular surgery have been shown to result in severe injury to the corneal endothelium in as little as twenty seconds. The turbulent flow of fluids may also cause tissue fragments to impact the delicate corneal endothelial cells or other intraocular tissues, thereby causing mechanical trauma to such tissues.

For further background regarding these problems, please refer to the following articles: Kim, et al., "Corneal endothelial damage by air bubbles during phacoemulsification", Archives of Ophthalmology, volume 115, pages 81-88, 1997; Beesley et al., "The effects of prolonged phacoemulsification time on the corneal endothelium", Annals of Ophthalmology, volume 18, no. 6, pages 216-219, 1986; Kondoh et al., "Quantitative measurement of the volume of air bubbles formed during ultrasonic vibration", Folia Ophthalmogica Japan, volume 45, no. 7, pages 718-720, 1994 and Kim et al., Investigative Ophthalmology & Visual Science, volume 37, no. 3, S84, 1996.

The fluid dynamics of intraocular irrigating solutions is also important during vitrectomy procedures and various other types of intraocular surgical procedures. Turbulence in intraocular fluids may also result from the movements of reciprocating vitrectomy handpieces, the alternating vacuum and irrigation modes of irrigation/aspiration handpieces and movements of other surgical handpieces and devices utilized in such procedures. The elimination or reduction of such turbulence helps to protect the retina and other tissues located in the posterior segment of the eye, as well as tissues located in the anterior segment of the eye, such as the corneal endothelial cells.

In view of these potential complications, there is a need for intraocular irrigating solutions having improved physical properties that: (1) reduce the potential for turbulence within the anterior and posterior chambers of the eye, (2) help to contain the movement of tissue fragments and air bubbles within the eye, and (3) facilitate the removal of lens fragments and other tissue fragments by making it easier for the surgeon to track the fragments with the tip of the surgical handpiece. The present invention is directed to fulfilling this need. Specifically, the present invention is directed to the provision of an irrigating solution that provides for greater control of the movement of tissue fragments, air bubbles and other particles during phacoemulsification, vitrectomy and other intraocular surgical procedures. This control of particle movement is fundamentally different from the above-discussed use of a layer of viscoelastic material to protect the corneal endothelial cells by means of a cushioning effect. The irrigating solution of the present invention is designed to provide a protective effect beyond that obtained by means of viscoelastic agents.

Assia et al. (1998) J. Cataract Refract. Surg. 24(1): 78-83 describes experimental studies on viscofluids for intraocular surgery.

GB 2,204,238 discloses viscoelastic fluids for intraocular surgery.

EP 517 970 Al discloses an additive for an irrigating solution or surgical solution. This document does not determine the viscosity of the disclosed solutions.

US 4,238,482 describes intraocular infusion irrigating solutions and methods of reducing stress-induced damage to lenses and corneal endothelia during surgical vitrectomy, irrigation, cataract aspiration, and phacoemulsification.

EP 938 903 A1 describes ophthalmic compositions with regulated viscosity that are applied to the external surface of the eye, particularly solutions for relieving dry eye symptoms and wetting contact lenses.

US 5,895,645 discloses artificial tear solutions that are applied topically to the cornea.

Huang et al. (2006) J. Vision 6:653-660 describes studies on accommodative microfluctuations and iris contour.

Silver and Quigley (2004) J. Glaucoma 13: 100-107 investigate aqueous flow through the iris-lens channel and provide estimates of differential pressure between the interior and posterior chambers.

Moses (1979) Am. J. Ophthalmol. 88: 585-591 investigates the circumferential flow in Schlemm's canal.

James et al. (1984) J. Phys. D: Appl. Phys. 17: 225-230 describes studies on viscosities of water between 0 and 60°C and selected aqueous sucrose solutions and discusses the data in relation to capillary viscometer calibration.

Beswick and McCulloch (1956) Brit. J. Ophthal. 40: 545-548 investigate the effect of hyaluronidase on the viscosity of the aqueous humor.

Fechner and Fechner (1983) Br. J. Ophthalmol. 67: 259-263 describe studies on lens implantation using methylcellulose.

Fleming et al. (1959) Arch. Ophthalmol. 61: 565-567 describe studies of the irritating action of methylcellulose.

Mueller and Deardorff (1956) J. Am. Pharm. A. (Scient. Ed.) 45: 334-341 investigate ophthalmic vehicles, in particular the effect of methylcellulose on the penetration of homatropine hydrobromide through the cornea.

### Summary of the Invention

The present invention is directed to the provision of intraocular irrigating solutions that help to prevent the risk of damage to intraocular tissues, while facilitating the efficiency of the surgical procedures. The irrigating solutions of the present invention are low viscosity solutions that exhibit less turbulence in the presence of phacoemulsification handpieces and other intraocular surgical devices. These solutions also restrain the movement of air bubbles and tissue fragments within the eye, and generally dampen the impact of ultrasonic handpieces, liquefracture handpieces, irrigation/aspiration handpieces, microscissors, vitrectomy handpieces and other surgical devices on intraocular tissues. The restrained movement of lens fragments within the eye protects ophthalmic tissues, and facilitates a more efficient surgical procedure by enabling the ophthalmic surgeon to locate and remove lens fragments more readily.

The intraocular irrigating solutions of the present invention have a viscosity greater than that of aqueous humor, but preferably have a surface tension similar to that of aqueous humor. Existing irrigating solutions generally have a viscosity similar to that of aqueous humor, but have surface tension higher than that of aqueous humor.

The present inventors have found that a slight enhancement of the viscosity of intraocular irrigating solutions greatly improves the ability of the solutions to protect intraocular tissues by containing the movement of tissue fragments and generally reducing the turbulence of the intraocular fluids, thereby making it easier for the fragments to be tracked and removed via aspiration. This slight enhancement of irrigating solution viscosity is also beneficial in vitrectomy procedures because it reduces the pulsatile movement of the retinal tissue and limits collateral tissue damage in the eye. The reduction of pulsatile movement of retinal tissue is particularly important in cases where the retina is partially detached.

The overall performance of the irrigating solutions of the present invention can be further enhanced by including an agent which reduces the surface tension to a level comparable to that of aqueous humor, thereby making the solutions more physiological.

### Brief Description of Drawings

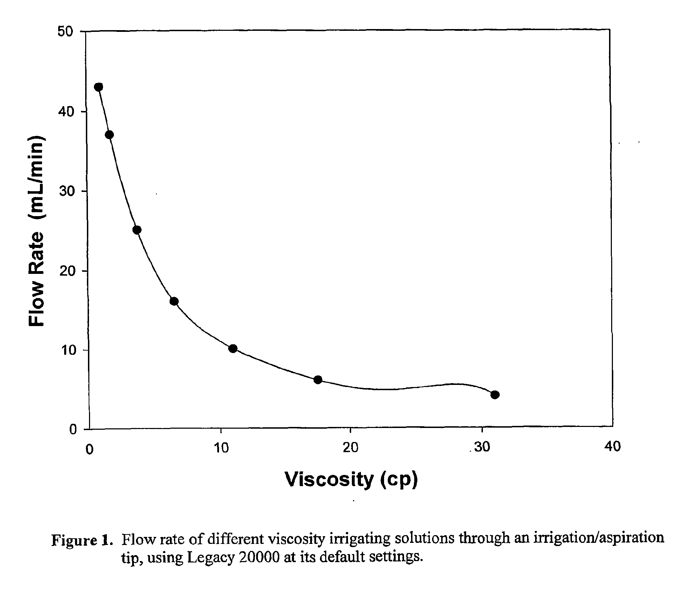

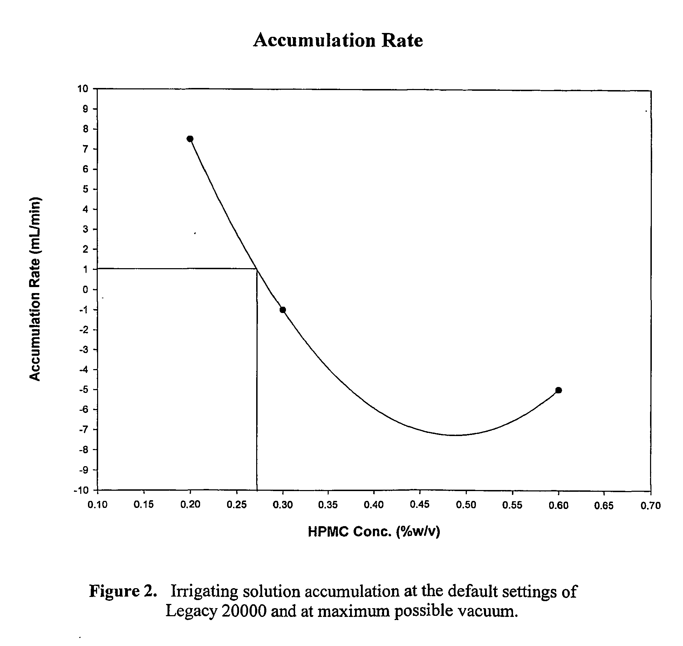

Figure 1 is a graph showing the effect of viscosity on flow rate; and Figure 2 is a graph showing the relationship between HPMC concentration and accumulation rate.

### Detailed Description of the Invention

The irrigating solutions of the present invention comprise a balanced salt solution and a viscosity-adjusting agent, said viscosity-adjusting agent consisting essentially of hydroxypropyl methycellulose at a concentration of 0.1 to 0.3 w/v %.

The electrolyte solution utilized in the present invention is a balanced salt solution, such as BSS^{™} (Balanced Salt Solution) Sterile Irrigating Solution manufactured by Alcon Laboratories, Inc., or BSS PLUS^{™} (Balanced Salt Solution) Sterile Irrigating Solution, also manufactured by Alcon Laboratories, Inc. However, the invention is not limited relative to the types of balanced salt solutions that may be utilized as a building block for the solutions of the present invention.

The agents utilized to adjust the viscosity of the electrolyte solution will comprise one compound that is compatible with intraocular tissues, namely hydroxypropyl methylcellulose ("HPMC"),

The following patent publications may be referred to for further details concerning viscosity-enhancing agents: U.S. Patent No. 4,861,760 (gellan gums); U.S. Patent No. 4,255,415 and WIPO Publication No. WO 94/10976 (polyvinyl alcohol); U.S. Patent No. 4,271,143 (carboxyvinyl polymers); WIPO Publication No. WO 99/51273 (xanthan gum); and WIPO Publication No. WO 99/06023 (galactomannans).

The above-described viscosity-adjusting agent will be utilized in an amount sufficient to provide the irrigating solutions of the present invention with an enhanced viscosity. As utilized herein, the phrase "enhanced viscosity" means a viscosity which is greater than the viscosity of aqueous humor and prior irrigating solutions, both of which generally have viscosities of approximately 1 mPa·s. The irrigating solutions of the present invention will typically have viscosities of from greater than 1 mPa·s to about 15 cps, preferably from about 2 to about 7 mPa·s.

The amount of viscosity adjusting agent utilized will vary depending on the degree of viscosity enhancement desired and the specific agent or agents selected. However, the concentration of the viscosity-adjusting agent in the irrigating solutions of the present invention will range from 0.1 to about 0.3 weight/volume percent ("w/v %") for HPMC.

It should be noted that it is necessary to achieve a balance between: (a) enhancing the viscosity of the solution, and (b) maintaining a solution viscosity that is acceptable for use with the irrigation/aspiration system employed during intraocular surgical procedures. Figure 1 of the accompanying drawings is a graph showing the flow rate of irrigating solutions of different viscosities through a normal irrigation/aspiration tip in the Series 20000 Legacy^{™} ("STTL") surgical operating system available from Alcon Laboratories, Inc. During generation of these data, all the settings on the STTL system were default instrumental settings. Figure 1 clearly shows the effect of increasing viscosity on flow rate of the irrigating solution, which is usually flowing under gravity.

During a surgical procedure, aspiration is carried out by applying vacuum through the tip of a surgical handpiece. Generally, the maximum vacuum or suction capability of the system is such that the irrigation rate is higher than the aspiration rate to maintain positive flow. Hence, the increase in viscosity of the irrigation solution should be such that the flow rate remains greater than the maximum aspiration rate. Figure 2 of the accompanying drawings illustrates this point.

Increasing the concentration of the viscosity-adjusting agent increases the viscosity of the solution, so at the same bottle height, the normal gravity fed irrigation flow rate of fluid into the eye decreases. As the net irrigation rate decreases, the effective aspiration rate, which is controlled independently by a peristaltic pump on the STTL, increases. Hence, the accumulation rate goes from a positive to a negative value. A minimum irrigation rate of 1 milliliter/minute of aspiration is needed to prevent drying up of the tissue. These competing factors must be balanced. In the case of HPMC, it has been determined that a HPMC concentration of 0.27 w/v% provides the desired level of viscosity enhancement without impeding normal irrigation and aspiration functions. It should be noted that this ideal concentration was determined using HPMC (E4M) in connection with the STTL surgical operating system and a standard phacoemulsification tip. The ideal concentration may vary somewhat, depending on the surgical operating system and phacoemulsification tip utilized.

The viscosity-adjusting agent is hydroxypropylmethylcellulose ("HPMC"). The present inventors have found that the addition of HPMC to a conventional balanced salt solution results in a significant reduction in turbulence during intraocular surgery, relative to the turbulence seen with the balanced salt solution alone. The preferred concentration of HPMC is about 0.2 to 0.3 w/v%, but this range may vary slightly depending on the particular ophthalmic surgical system being utilized and the instrument settings of that system. Irrigating solutions containing this concentration of HPMC will have a viscosity of about 4 to 6 mPa·s. The most preferred viscosity-adjusting agent is HPMC (E4M) at a concentration of 0.22 to 0.27 w/v%.

As indicated above, the irrigating solutions of the present invention preferably also include an agent to modify the surface tension of the solutions so as to resemble the surface tension of the aqueous humor. The surface tension of the aqueous humor is approximately 50 mN/m. The irrigating solutions of the present invention will therefore preferably have a surface tension in the range of 40 to 60 mN/m or somewhat less.

It should be noted here that viscosity can be increased by an appropriate agent without affecting surface tension, and that surface tension can be reduced to the level of aqueous/vitreous humor by inclusion of an appropriate surface-active agent independent of viscosity. Thus, these two physical properties of irrigating solutions are independent of each other. However, in some cases, the viscosity-adjusting agent may also function as the surface tension reducing agent. This is true with respect to the preferred embodiment of the present invention, wherein HPMC is utilized both as a viscosity-adjusting agent and a surface tension reducing agent.

In other cases, it may be necessary to add a separate agent to the irrigating solution for purposes of reducing the surface tension of the solution. Possible agents which can be utilized for this purpose include: Polyoxyl 35 castor oil (Cremophore^{™} EL and Cremophore^{™} EL-P, available from BASF Corp.), Polyoxyl 40 Hydrogenated Castor Oil (HCO-40), Solutol^{™} HS 15 (BASF Corp.), Polysorbate 80, Tocophersolan (TPGS), and other ophthalmically acceptable surface active agents.

The following examples are provided to further illustrate various features of the present invention.

### Example 1

| **Component** | **Amount (w/v%)** | **Function** |
|---|---|---|
| HPMC (E4M) | 0.1 to 0.3 | Viscosity and Surface Tension Modifier |
| Sodium Chloride | 0.744 | Tonicity Agent |
| Potassium Chloride | 0.0395 | Essential Ion |
| Dibasic Sodium Phosphate (Anhydrous) | 0.0433 | Buffering Agent |
| Sodium Bicarbonate | 0.219% + 20% xs | Physiological Buffer |
| Hydrochloric Acid | Adjust pH | pH Adjust |
| Sodium Hydroxide | Adjust pH | pH Adjust |
| Water for Injection | 100% | Vehicle |

The above-described formulation may be prepared as follows: First, the water for injection is brought close to boiling or at boiling. The HPMC is then slowly added to the water under continuous stirring to thoroughly disperse it in the water. Then the mixture is slowly allowed to cool, stirring continuously. Once at room temperature, the mixture should start clearing up. Then the mixture is stored overnight in an appropriate container to fully hydrate the HPMC. The following day, the remaining ingredients are added to the HPMC solution, additional water for injection is added if needed to bring the solution to final volume, and the final solution is filtered, packaged in bottles and autoclaved.

### Example 2

| **Component** | **Amount (w/v%)** | **Function** |
|---|---|---|
| HPMC (E4M) | 0.1 to 0.3 | Viscosity and Surface Tension Modifier |
| Sodium Chloride | 0.64 | Tonicity Agent |
| Potassium Chloride | 0.075 | Essential Ion |
| Calcium Chloride (Dihydrate) | 0.048 | Essential Ion |
| Magnesium Chloride (Hexahydrate) | 0.03 | Essential Ion |
| Sodium Acetate (Trihydrate) | 0.039 | Buffering Agent |
| Sodium Citrate (Dihydrate) | 0.17 | Buffering Agent |
| Hydrochloric Acid | Adjust pH | pH Adjust |
| Sodium Hydroxide | Adjust pH | pH Adjust |
| Water for Injection | 100% | Vehicle |

The above-described formulation may be prepared by means of the method described in Example 1, above.

### Example 3

Three solutions were prepared and tested to evaluate the physical properties of the solutions of the present invention versus related solutions. The solutions tested and the respective physical properties of the solutions were as follows:

| **Solution** | **Osmolality** **mOsm/kg** | **Viscosity** **mPa·s** | **Surface Tension** **mN/m** |
|---|---|---|---|
| BSS* | 304,305 | 1.02, 1.06 | 70, 73 |
| BSS + 0.05% cremophor | 305, 305 | 0.99, 1.01 | 43, 43 |
| BSS + 0.3% HPMC (grade E4M) | 320, 322 | 6.9, 7.0 | 48, 49 |

| | | | |
|---|---|---|---|
| *As utilized in the above table, the term "BSS" refers to BSS™ (Balanced Salt Solution) Sterile Irrigating solution manufactured by Alcon Laboratories, Inc., Fort Worth, Texas. | | | |

As indicated above, the addition of 0.3% HPMC to the BSS solution increased the viscosity from approximately 1 mPa·s to 7 mPa·s, and reduced the surface tension from approximately 71.5 mN/m to approximately 48.5 mN/m. Thus, the addition of this amount of HPMC increased the viscosity of the balanced salt solution and reduced its surface tension, in accordance with the basic principles of the present invention. Conversely, the addition of 0.05% cremophor to the balanced salt solution had no effect on viscosity, but reduced the surface tension of the balanced salt solution from approximately 71.5 mN/m to 43 mN/m.

The above-identified solutions were tested in a simulated intraocular surgery model to determine if the addition of cremophor and HPMC to the balanced salt solution affected the performance of the solution relative to the turbulence of the solution during intraocular surgical procedures. It was determined that the addition of cremophor to the balanced salt solution, although effective in reducing the surface tension of the solution, had little if any effect on the performance of the balanced salt solution. However, the solution containing HPMC demonstrated much less turbulence than the balanced salt solution alone. This turbulence was judged based on the movement of air bubbles and the movement of lens fragments.

The spinning and rotation of lens fragments seen with the balanced salt solution alone was reduced significantly by the inclusion of HPMC in the solution. The dampening of the movement of the lens particles facilitated an easier removal of the particles from the eye during the simulated surgical procedure. This dampening effect facilitated a more efficient surgical procedure and reduced the time required for the procedure.

Conversely, there appeared to be no difference between the balanced salt solution alone and the balanced salt solution containing cremophor with regard to bubble formulation, rate of flow or the visual hydrodynamics of the irrigating solutions.

The foregoing results confirm that the addition of a small amount of a viscosity enhancing agent reduces the turbulence of intraocular fluids during surgical procedures, dampens the movement of bubbles and lens fragments, and generally renders the procedure more efficient.

## Claims

1. An ophthalmic pharmaceutical solution for irrigating ocular tissues during an intraocular surgical procedure comprising a balanced salt solution and a viscosity-adjusting agent, said viscosity-adjusting agent consisting essentially of hydroxypropyl methylcellulose at a concentration of 0.1 to 0.3 w/v %.

2. The solution according to claim 1, wherein the concentration of hydroxypropyl methylcellulose in the solution is 0.2 to 0.3 w/v %.

3. The solution according to claim 1 or 2, wherein the balanced salt solution is an electrolyte/nutrient solution.

4. The solution according to any one of the preceding claims, said solution having a viscosity greater than the viscosity of aqueous humor.

5. The solution according to claim 4, wherein the viscosity of the solution reduces the turbulence of the solution during an intraocular surgical procedure.

6. The solution according to any one of the preceding claims, wherein said hydroxypropyl methylcellulose is hydroxypropyl methylcellulose E4M.

7. The solution according to any one of the preceding claims, wherein the hydroxypropyl methylcellulose reduces the surface tension of the solution.

8. The solution according to any one of the preceding claims, wherein the surface tension of the solution is similar to that of aqueous humor.

9. The use of an irrigating solution according to claim 1 for the manufacture of a pharmaceutical solution for irrigating intraocular tissues during an ophthalmic surgical procedure, wherein the viscosity of the solution reduces the turbulence of the solution during the surgical procedure.

10. The use according to claim 9, wherein the concentration of hydroxypropyl methylcellulose in the solution is 0.2 to 0.3 w/v %.

11. The use according to claim 9 or 10, wherein the irrigating solution is an electrolyte/nutrient solution.

12. The use according to any one of claims 9 to 11, wherein the solution has a viscosity greater than the viscosity of aqueous humor.

13. The use according to any one of claims 9 to 12, wherein said hydroxypropyl methylcellulose is hydroxypropyl methylcellulose E4M.

14. The use according to any one of claims 9 to 13, wherein the hydroxypropyl methylcellulose reduces the surface tension of the solution.

15. The use according to any one of claims 9 to 14, wherein the surface tension of the solution is similar to that of aqueous humor.

## Patentansprüche

1. Ophthalmische pharmazeutische Lösung zum Spülen von okularem Gewebe während eines intraokularen chirurgischen Eingriffs, umfassend eine ausgeglichene Salzlösung und ein Viskositätseinstellmittel, wobei das Viskositätseinstellmittel im Wesentlichen aus Hydroxypropylmethylcellulose in einer Konzentration von 0,1 bis 0,3 % Gew./Vol. besteht.

2. Lösung nach Anspruch 1, wobei die Konzentration an Hydroxypropylmethylcellulose in der Lösung 0,2 bis 0,3 % Gew./Vol. beträgt.

3. Lösung nach Anspruch 1 oder 2, wobei die ausgeglichene Salzlösung eine Elektrolyt/Nährstoff-Lösung ist.

4. Lösung nach einem der vorstehenden Ansprüche, wobei die Lösung eine höhere Viskosität als Kammerwasser hat.

5. Lösung nach Anspruch 4, wobei die Viskosität der Lösung die Turbulenz der Lösung während eines intraokularen chirurgischen Eingriffes reduziert.

6. Lösung nach einem der vorstehenden Ansprüche, wobei die Hydroxypropylmethylcellulose Hydroxypropylmethylcellulose E4M ist.

7. Lösung nach einem der vorstehenden Ansprüche, wobei die Hydroxypropylmethylcellulose die Oberflächenspannung der Lösung verringert.

8. Lösung nach einem der vorstehenden Ansprüche, wobei die Lösung eine ähnliche Oberflächenspannung wie Kammerwasser hat.

9. Verwendung einer Spüllösung nach Anspruch 1 zur Herstellung einer pharmazeutischen Lösung zum Spülen von intraokularem Gewebe während eines ophthalmischen chirurgischen Eingriffes, wobei die Viskosität der Lösung die Turbulenz der Lösung während des chirurgischen Eingriffes verringert.

10. Verwendung nach Anspruch 9, wobei die Hydroxypropylmethylcellulosekonzentration in der Lösung 0,2 bis 0,3 % Gew./Vol. beträgt.

11. Verwendung nach Anspruch 9 oder 10, wobei die Spüllösung eine Elektrolyt/NährstoffLösung ist.

12. Verwendung nach einem der Ansprüche 9 bis 11, wobei die Lösung eine höhere Viskosität als Kammerwasser hat.

13. Verwendung nach einem der Ansprüche 9 bis 12, wobei die Hydroxypropylmethylcellulose Hydroxypropylmethylcellulose E4M ist.

14. Verwendung nach einem der Ansprüche 9 bis 13, wobei die Hydroxypropylmethylcellulose die Oberflächenspannung der Lösung verringert.

15. Verwendung nach einem der Ansprüche 9 bis 14, wobei die Lösung eine ähnliche Oberflächenspannung wie Kammerwasser hat.

## Revendications

1. Solution pharmaceutique ophtalmique pour l'irrigation de tissus oculaires pendant une intervention chirurgicale intraoculaire comprenant une solution saline équilibrée et un agent de réglage de la viscosité, ledit agent de réglage de la viscosité étant essentiellement constitué d'hydroxypropyl méthylcellulose à une concentration de 0,1 à 0,3% poids/volume.

2. Solution selon la revendication 1, dans laquelle la concentration d'hydroxypropyl méthylcellulose dans la solution va de 0,2 à 0,3% poids/volume.

3. Solution selon la revendication 1 ou 2, dans laquelle la solution saline équilibrée est une solution électrolyte/nutriment.

4. Solution selon l'une quelconque des revendications précédentes, ladite solution présentant une viscosité plus grande que la viscosité de l'humeur aqueuse.

5. Solution selon la revendication 4, dans laquelle la viscosité de la solution réduit la turbulence de la solution pendant une intervention chirurgicale intraoculaire.

6. Solution selon l'une quelconque des revendications précédentes, dans laquelle ladite hydroxypropyl méthylcellulose est l'hydroxypropyl méthylcellulose E4M.

7. Solution selon l'une quelconque des revendications précédentes, dans laquelle l'hydroxypropyl méthylcellulose réduit la tension superficielle de la solution.

8. Solution selon l'une quelconque des revendications précédentes, dans laquelle la tension superficielle de la solution est semblable à celle de l'humeur aqueuse.

9. Utilisation d'une solution d'irrigation selon la revendication 1 pour la fabrication d'une solution pharmaceutique pour l'irrigation de tissus intraoculaires pendant une intervention chirurgicale ophtalmique, dans laquelle la viscosité de la solution réduit la turbulence de la solution pendant l'intervention chirurgicale.

10. Utilisation selon la revendication 9, dans laquelle la concentration d'hydroxypropyl méthylcellulose dans la solution va de 0,2 à 0,3% poids/volume.

11. Utilisation selon la revendication 9 ou 10, dans laquelle la solution d'irrigation est une solution électrolyte/nutriment.

12. Utilisation selon l'une quelconque des revendications 9 à 11, dans laquelle la solution a une viscosité plus grande que la viscosité de l'humeur aqueuse.

13. Utilisation selon l'une quelconque des revendications 9 à 12, dans laquelle ladite hydroxypropyl méthylcellulose est l'hydroxypropyl méthylcellulose E4M.

14. Utilisation selon l'une quelconque des revendications 9 à 13, dans laquelle l'hydroxypropyl méthylcellulose réduit la tension superficielle de la solution.

15. Utilisation selon l'une quelconque des revendications 9 à 14, dans laquelle la tension superficielle de la solution est semblable à celle de l'humeur aqueuse.
